(19) Europäisches Patentamt
European Patent Office
Office européen des brevets

(11) Veröffentlichungsnummer: **0 349 483 B1**

(12) # EUROPÄISCHE PATENTSCHRIFT

(45) Veröffentlichungstag der Patentschrift: **09.02.94**

(51) Int. Cl.⁵: **C07C 323/12**, //C10N30/00, C10N30/06,C10N40/08, C10N40/20,C10N40/00

(21) Anmeldenummer: **89810429.4**

(22) Anmeldetag: **07.06.89**

Die Akte enthält technische Angaben, die nach dem Eingang der Anmeldung eingereicht wurden und die nicht in dieser Patentschrift enthalten sind.

(54) **Zusätze für funktionelle Flüssigkeiten.**

(30) Priorität: **13.06.88 CH 2258/88**
**16.05.89 CH 1808/89**

(43) Veröffentlichungstag der Anmeldung:
**03.01.90 Patentblatt 90/01**

(45) Bekanntmachung des Hinweises auf die Patenterteilung:
**09.02.94 Patentblatt 94/06**

(84) Benannte Vertragsstaaten:
**BE DE ES FR GB IT NL**

(56) Entgegenhaltungen:
**EP-A- 0 166 696**
**EP-A- 0 237 489**

(73) Patentinhaber: **CIBA-GEIGY AG**
**Klybeckstrasse 141**
**CH-4002 Basel(CH)**

(72) Erfinder: **Wirth, Hermann O., Dr.**
**Lessingstrasse 24**
**D-6140 Bensheim 3(DE)**
Erfinder: **Müller, Klaus, Dr.**
**Am Stadtgraben 7**
**D-7850 Lörrach(DE)**
Erfinder: **Friedrich, Hans-Helmut**
**Am Rauhenstein 8**
**D-6147 Lautertal 2(DE)**

EP 0 349 483 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue thioäthergruppenhaltige Verbindungen und funktionelle Flüssigkeiten aus der Reihe der Schmierstoffe, wie Schmieröle und Schmierfette, Hydraulikflüssigkeiten und Metallbearbeitungsflüssigkeiten, enthaltend thioäthergruppenhaltige Verbindungen und die Verwendung dieser Verbindungen als Additive.

Funktionellen Flüssigkeiten werden im allgemeinen verschiedene Zusatzstoffe zur Verbesserung ihrer Gebrauchseigenschaften beigegeben. Da beispielsweise Schmieröle Zur Uebertragung grösserer Kräfte ein hohes Lasttragevermögen benötigen, werden diesen sogenannte Hochdruck- und Antiverschleiss-Additive zugesetzt, wodurch die sonst auftretenden Verschleisserscheinungen stark erniedrigt werden. Wenn andererseits z.B. Sauerstoff und Feuchtigkeit gleichzeitig auf eine Metalloberfläche einwirken, kann Korrosion auftreten, weshalb Korrosionsinhibitoren zugegeben werden. Die beispielsweise bei erhöhter Temperatur verstärkt durch Luftsauerstoff eintretenden Oxidationsreaktionen in einem Schmierstoff können durch Zugabe von Antioxidantien unterbunden werden. Es ist bekannt, dass bestimmte Stoffe als Additive für Schmieröle eine Anzahl derartiger Eigenschaften in sich vereinigen können; sie werden als sogenannte Vielzweck-Additive bezeichnet. Solche Stoffe sind natürlich aus ökonomischen und praktischen Gründen sehr gefragt.

Aus der US-PS 4 246 127 sind beispielsweise Di- und Polysulfide und deren Einsatz als Schmierstoffzusätze bekannt. Aus der EP-A 0 166 696 sind weiter thioäthergruppenhaltige Verbindungen und Zusammensetzungen derselben mit Schmierstoffen und Hydraulikflüssigkeiten bekannt.

Es wurden nun thioäthergruppenhaltige Verbindungen gefunden, die in Zusammensetzungen mit Schmierstoffen, Hydraulikflüssigkeiten oder Metallbearbeitungsflüssigkeiten ganz herausragende Eigenschaften aufweisen und die beispielsweise in Schmierölen sich in ihrem Wirkungsverhalten deutlich gegenüber vorbeschriebenen Verbindungen abheben.

Die vorliegende Erfindung betrifft Verbindungen der Formel

$$R^1-S-CH_2-\underset{OH}{CH}-CH_2-Q-CH_2-\underset{OH}{CH}-CH_2-S-R^2 \quad (I),$$

wobei Q -S-, -S-S-, -S-CH$_2$-S- oder -S-CH$_2$-CH$_2$-S- bedeutet, und
$R^1$ und $R^2$ tert.-$C_4$- bis tert.-$C_7$-Alkyl bedeuten.

Vorzugsweise sind in der Formel I $R^1$ und $R^2$ tert.-Butyl.

Besonders bevorzugte Verbindungen sind

$$\text{tert.-Butyl}-S-CH_2-\underset{OH}{CH}-CH_2-S-CH_2-\underset{OH}{CH}-CH_2-S-\text{tert.-Butyl} \quad (III),$$

$$\text{tert.-Butyl}-S-CH_2-\underset{OH}{CH}-CH_2-S-S-CH_2-\underset{OH}{CH}-CH_2-S-\text{tert.-Butyl} \quad (IV),$$

$$\text{tert.-Butyl}-S-CH_2-\underset{OH}{CH}-CH_2-S-CH_2-S-CH_2-\underset{OH}{CH}-CH_2-S-\text{tert.-Butyl} \quad (V),$$

$$\text{tert.-Butyl}-S-CH_2-\underset{OH}{CH}-CH_2-S-CH_2-CH_2-S-CH_2-\underset{OH}{CH}-CH_2-S-\text{tert.-Butyl} \quad (VI),$$

Die vorliegende Erfindung betrifft auch Zusammensetzungen, enthaltend

a) eine funktionelle Flüssigkeit aus der Reihe der Schmierstoffe, Hydraulikflüssigkeiten und Metallbearbeitungsflüssigkeiten und

b) wenigstens eine Verbindung der Formel (I).

Insbesondere betrifft vorliegende Erfindung Zusammensetzungen, enthaltend

a) ein Schmieröl aus der Reihe der Mineralöle, der synthetischen Oele oder der Mischungen davon, und

b) wenigstens eine Verbindung der Formel I.

Vorzugsweise enthalten die Zusammensetzungen wenigstens eine Verbindung der Formel I, worin $R^1$ und $R^2$ gleich tert.-Butyl ist.

Besonders bevorzugte Zusammensetzungen enthalten wenigstens eine der Verbindungen der Formeln

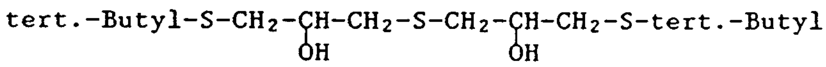
(III),

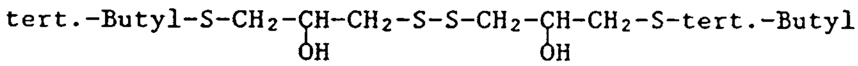
(IV),

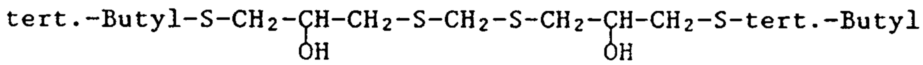
(V),

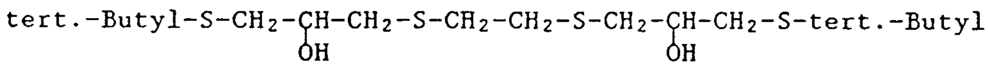
(VI),

$R^1$ und $R^2$ in Formel I stellen unabhängig voneinander eine tertiäre Alkylgruppe mit 4 bis 7 C-Atomen dar und es kann sich beispielsweise um eine tert.-Butyl- oder um eine tert.- Pentyl-, Hexyl- oder Heptylgruppe handeln.

Die Herstellung der Verbindungen nach vorliegender Erfindung erfolgt nach an sich bekannter Weise.

Die Herstellung der als Zwischenprodukt für die Verbindungen der Formel I dienenden Alkyl-thiaglycidyläther geschieht auf folgende Weise:

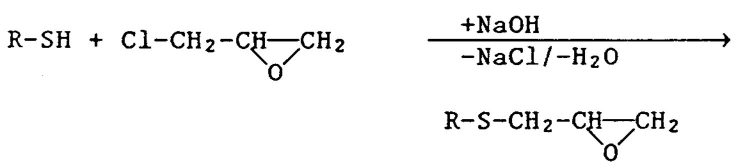

(II),

wobei der Substituent R die bereits angegebene Bedeutung für $R^1$ oder $R^2$ hat. Besonders vorteilhaft für diese Umsetzung ist die Verwendung eines Phasentransfer-Katalysators, wie z.B. Tetrabutylammoniumchlorid. Die Herstellung von Alkyl-thiaglycidylethern ist auch in US-A-2 965 652, US-A-2 731 437 und BE-A-609 375 beschrieben.

Die Alkyl-thiaglycidylether der Formel II lassen sich dann beispielsweise mit Natriumhydrogensulfid zu Verbindungen vom Typ der Formel III umsetzen. Die nach Addition von $H_2S$ an Verbindungen der Formel II gebildeten Mercaptane können z.B. mit Dimethylsulfoxid in Verbindungen vom Typ der Formel IV übergeführt werden. Die genannten Mercaptane liefern mit Formaldehyd Verbindungen vom Typ der Formel V während die Addition von Verbindungen vom Typ der Formel II an 1,2-Dimercaptoethan Verbindungen vom Typ der Formel VI liefert.

Die Verbindungen der Formel I wirken schon in sehr geringen Mengen als Additive in funktionellen Flüssigkeiten, wie Schmieröle, Hydraulikflüs-sigkeitenund Metallbearbeitungsflüssigkeiten. Sie werden den funktionellen Flüssigkeiten zweckmässig in einer Menge von 0,01 bis 5 Gew.-%, vorzugsweise in einer Menge von 0,05 bis 3 Gew.-%, bezogen auf die funktionelle Flüssigkeit, zugesetzt.

Die in Frage kommenden Schmieröle sind dem Fachmann geläufig und z.B. in Dieter Klamann "Schmierstoffe und verwandte Produkte" (Verlag Chemie, Weinheim, 1982) oder in Schewe-Kobek, "Das Schmiermittel-Taschenbuch", Dr. Alfred Hüthig-Verlag, Heidelberg, 1974, beschrieben. Besonders geeignet sind neben Mineralölen z.B. Poly-α-Olefine, Schmierstoffe auf Esterbasis, Phosphate, Glykole, Polyglykole und Polyalkylenglykole.

Die Verbindungen der Formel I sind gut in Schmierstoffen löslich, sind deshalb als Zusätze beispielsweise zu Schmierölen und Schmierfetten besonders geeignet und führen zu einer Verbesserung der Hochdruck- und Antiverschleiss-Eigenschaften. Ebenso ist auch auf ihre antioxidative und antikorrosive Wirkung auch gegenüber Kupfer hinzuweisen. Darüberhinaus werden die gängigen Dichtungsmaterialien nicht angegriffen. Ueberraschenderweise ist schliesslich die Herstellung von sogenannten Masterbatches möglich.

Das Vorliegen von Verbindungen der Formel I in Schmierstoffen ermöglicht es, aschebildende Additive, also insbesondere Zinkverbindungen oder phosphorhaltige Additive, vornehmlich Zinkdithiophosphate, wenigstens teilweise zu ersetzen. Zink und seine Verbindungen und gerade Phosphor und seine Verbindungen sind als Bestandteil eines Additives, das in einem Motorenschmieröl in Benzinmotoren zur Anwendung gelangt, im Hinblick auf eine mögliche Desaktivierung des heute einem benzingetriebenen Motor nachgeschalteten Abgas-Katalysators durch die mit dem Abgas entweichenden Zink- und Phosphorverbindungen in ihrer Menge zu beschränken oder bevorzugt ganz wegzulassen.

Im Gegensatz zur möglichen Schädigung des Katalysators an benzingetriebenen Motoren durch die bisherigen Additive, ist durch die Verbindungen der Formel I nach vorliegender Erfindung eine weitergehende Phosphorreduzierung und damit eine gesteigerte Betriebssicherheit des Katalysators möglich.

Es wurden auch neue Verbindungen und daraus neue Zusammensetzungen funktioneller Flüssigkeiten und dabei insbesondere Schmierölzusammensetzungen gefunden, die weiter verbesserte Eigenschaften gegenüber den bisher bekannten Produkten aufweisen. Die Produkte zeichnen sich durch hohe Stabilität gegenüber oxidativer Degradation und solvolytischer Spaltbarkeit aus und verleihen beispielsweise den Schmierölzusammensetzungen einen überraschenden Verschleissschutz, der insbesondere am Ventiltrieb, also an den Nocken der Nockenwellen und den Stösseln von Verbrennungskraftmaschinen nach dem Diesel- und Ottoprinzip wirksam wird. Darüberhinaus weisen die Verbindungen der Formel I und diese Verbindungen enthaltende Zusammensetzungen eine gute Dichtungsverträglichkeit auf und wirken nicht korrosiv gegenüber Kupfer.

Die erfindungsgemässen Zusammensetzungen sind zur Verwendung als Schmieröl-Zusammensetzungen für Verbrennungskraftmaschinen im allgemeinen, für Motoren nach dem Dieselprinzip im besonderen und für Motoren nach dem Ottoprinzip ganz besonders bevorzugt, geeignet. Unter der Bezeichnung Ottoprinzip werden die fremdgezündeten Verbrennungskraftmaschinen nach dem Hubkolbenprinzip verstanden. Die Verbrennungskraftmaschinen können den bekannten Zwecken dienen, also beispielsweise zur Verwendung als Stationärmotoren oder als Antriebe in Verkehrsmitteln, wie Schiffen, Flugzeugen und insbesondere in Kraftfahrzeugen.

Die Erfindung kann beispielsweise Zusammensetzungen betreffen, worin das Schmieröl ein Oel für Verbrennungskraftmaschinen nach dem Diesel- oder Ottoprinzip ist.

Die vorliegende Erfindung betrifft daher auch die Verwendung von Verbindungen der Formel I als Vielzweck-Additive für Schmierstoffe, Hydraulikflüssigkeiten oder Metallbearbeitungsflüssigkeiten. Zweckmässig werden Verbindungen der Formel I als Vielzweck-Additive in Schmierölen verwendet. Bevorzugt finden Verbindungen der Formel I als Hochdruck- und Verschleissschutzadditive in Schmierstoffen und insbesondere Schmierölen Verwendung.

Die erfindungsgemässen Zusammensetzungen betreffen zweckmässig auch aschearme oder aschefreie und phosphorfreie Zusammensetzungen enthaltend (a) ein Mineralöl oder ein synthetisches Oel oder eine Mischung davon oder eine Hydraulikflüssigkeit oder eine Metallbearbeitungsflüssigkeit und (b) wenigstens eine Verbindung der Formel (I).

Die vorliegende Erfindung betrifft daher auch die Verwendung von Verbindungen der Formel I als phosphorfreie Hochdruck- und Verschleissschutzadditive für Schmierstoffe, Hydraulikflüssigkeiten oder Metallbearbeitungsflüssigkeiten.

Die Erfindung betrifft insbesondere Zusammensetzungen der vorbeschriebenen Art der API-Klassifikation SF, SG, CD und/oder CE oder der CCMC-Klassen G 1, G 2, G 3, D 1, D 2, D 3 und/oder PD 1.

Demnach stellen die Zusammensetzungen zweckmässig Motorenöle für Kraftfahrzeuge, und dabei im wesentlichen für Personenkraftwagenmotoren und Nutzfahrzeugmotoren, dar, die in der API-Klassifikation (American Petroleum Institute) wenigstens einer der Kategorien mit den Kennbuchstaben SF, SG, CD oder CE und in der CCMC-Klassifikation (Committee of Common Market Automobile Constructors) wenigstens einer der Klassen G 1, G 2, G 3, D 1, D 2, D 3 oder PD 1 entsprechen.

Die Erfindung umfasst auch ein Verfahren zur Verminderung des Verschleisses in Verbrennungskraftmaschinen nach dem Diesel- oder Ottoprinzip und insbesondere an den Nocken der Nockenwellen und an den Stösseln des Ventiltriebes, das dadurch gekennzeichnet ist, dass der Schmierölkreislauf der Verbrennungskraftmaschine mit einer Zusammensetzung oben beschriebener Art betrieben wird.

Die Erfindung umfasst auch ein Verfahren zur Vermeidung der Desaktivierung des einer benzingetriebenen Verbrennungskraftmaschine nachgeschalteten Abgaskatalysators, das dadurch gekennzeichnet ist, dass der Schmierölkreislauf der Verbrennungskraftmaschine mit einer Zusammensetzung oben beschriebener Art betrieben wird.

Die Schmierstoffe können zusätzlich andere Additive enthalten, die zugegeben werden, um die Grundeigenschaften von Schmierstoffen noch weiter zu verbessern; dazu gehören: Antioxidantien, Metallpassivatoren, Korrosionsinhibitoren, Viskositätsindex-Verbesserer, Stockpunkterniedriger, Dispergiermittel,

Detergentien, sowie weitere Hochdruck-Zusätze und Antiverschleiss-Additive. Die genannten Vorbehalte gegenüber ascheproduzierenden, insbesondere Zinkverbindungen, und gegenüber Phosphorverbindungen gelten sinngemäss auch für die zusätzlichen Additive, wobei ein gewisser Gehalt an zinkhaltigen Phosphor/Schwefel-Verbindungen toleriert wird.

Beispiele für phenolische Antioxidantien

1. Alkylierte Monophenole
2,6-Di-tert-butyl-4-methylphenol, 2,6-Di-tert-butylphenol, 2-tert-Butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, o-tert-Butylphenol.

2. Alkylierte Hydrochinone
2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol.

3. Hydroxylierte Thiodiphenylether
2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol).

4. Alkyliden-Bisphenole
2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-oder -5-iso-butylphenol), 2,2'-Methylen-bis-[6-(α-methylbenzyl)-4-nonylphenol], 2,2'-Methylen-bis-[6-(α,α-di-methylbenzyl)-4-nonylphenol], 4,4'-Methylen-bis-(2,6-di-tert-butyl-phenol), 4,4'-Methylen-bis-(6-tert-butyl-2-methylphenol), 1,1-Bis-(5-tert-butyl-4-hydroxy-2-methylphenyl)-butan, 2,6-Di-(3-tert-butyl-5-methyl-2-hydroxybenzyl)-4-methylphenol, 1,1,3-Tris-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methylphenyl)-dicyclopentadien, Bis-[2-(3'-tert-butyl-2'-hydroxy-5'-methyl-benzyl)-6-tert-butyl-4-methyl-phenyl]-terephthalat.

5. Benzylverbindungen
1,3,5-Tri-(3,5-di-tert-butyl-4-hydroxybenzyl)-2,4,6-trimethylbenzol, Bis-(3,5-di-tert-butyl-4-hydroxybenzyl)-sulfid, 3,5-Di-tert-butyl-4-hydroxybenzyl-mercaptoessigsäure-isooctylester, Bis-(4-tert-butyl-3-hydroxy-2,6-dimethylbenzyl)-dithiol-terephthalat, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat,3,5-Di-tert-butyl-4-hydroxy-benzyl-phosphonsäure-dioctadecylester, 3,5-Di-tert-butyl-4-hydroxybenzyl-phosphonsäure-monoethylester, Calcium-salz.

6. Acylaminophenole
4-Hydroxy-laurinsäureanilid, 4-Hydroxy-stearinsäureanilid, 2,4-Bis-octyl-mercapto-6-(3,5-di-tert-butyl-4-hydroxyanilino)-s-triazin, N-(3,5-di-tert-butyl-4-hydroxyphenyl)-carbaminsäureoctylester.

7. Ester der β-(3,5-Di-tert-butyl-4-hydroxyphenyl)-propionsäure
mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Bis-hydroxyethyl-oxalsäurediamid.

8. Ester der β-(5-tert-butyl-4-hydroxy-3-methylphenyl)-propion säure
mit ein- oder mehrwertigen Alkoholen, wie z.B. mit Methanol, Diethylenglycol, Octadecanol, Triethylenglycol, 1,6-Hexandiol, Pentaerythrit, Neopentylglycol, Tris-hydroxyethyl-isocyanurat, Thiodiethylenglycol, Di-hydroxyethyl-oxalsäurediamid.

9. Amide der β-(3,5-Di-tert-butyl-4-hydroxyphenyl)-propionsäure,
wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

Beispiele für aminische Antioxidantien:

N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxy-phenyl)-amin, 2,6-Di-tert-butyl-4-dimethylamino-methyl-phenol, 2,4'-Di-amino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino)-propan, (o-Tolyl)-biguanid, Di-[4-(1',3'-di-methyl-butyl)-phenyl]amin, tert-octyliertes N-Phenyl-1-naphthylamin, Gemisch aus mono- und dialkylierten tert-Butyl-/tert-Octyldiphenylaminen, 2,3-Dihydro-3,3-dimethyl-4H-1,4-benzothiazin, Phenothiazin, N-Allyl-phenothiazin.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure.

Beispiele für Metall-Desaktivatoren, z.B. für Kupfer, sind:

Triazole, Benztriazole und deren Derivate, Tolutriazole und deren Derivate, 2-Mercaptobenzthiazol, 2-Mercaptobenztriazol, 2,5-Dimercaptobenztriazol, 2,5-Dimercaptobenzthiadiazol, 5,5′-Methylenbisbenztriazol, 4,5,6,7-Tetrahydrobenztriazol, Salicyliden-propylendiamin, Salicylaminoguanidin und dessen Salze.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Alkenylbernsteinsäureanhydrid, z.B. Dodecenylbernsteinsäure-anhydrid, Alkenylbernsteinsäure-Teilester und -Teilamide, 4-Nonylphenoxy-essigsäure.
b) Stickstoffhaltige Verbindungen, z.B.:
    I. Primäre, sekundäre oder tertiäre aliphatische oder cycloaliphatische Amine und Amin-Salze von organischen und anorganischen Säuren, z.B. öllösliche Alkylammoniumcarboxylate.
    II. Heterocyclische Verbindungen, z.B.:
    Substituierte Imidazoline und Oxazoline.
c) Phosphorhaltige Verbindungen, z.B.:
Aminsalze von Phosphorsäurepartialestern oder Phosphonsäurepartialestern, Zinkdialkyldithiophosphate.
d) Schwefelhaltige Verbindungen, z.B.:
Barium-dinonylnaphthalin-sulfonate, Calciumpetroleum-sulfonate.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

Beispiele für Verschleissschutz-Additive sind:

Schwefel und/oder Phosphor und/oder Halogen enthaltende Verbindungen, wie geschwefelte pflanzliche Oele, Zinkdialkyldithiophosphate, Tritolylphosphat, chlorierte Paraffine, Alkyl- und Aryldi- und tri-sulfide, Triphenylphosphorothionate, Diethanolaminomethyltolyltriazol, Di(2-ethylhexyl)aminomethyltolyltriazol.

Die nachfolgenden Beispiele erläutern die Erfindung weiter. Teile- und Prozentangaben beziehen sich dabei auf das Gewicht, sofern nichts anderes angegeben ist.

Beispiel 1:

Herstellung von:

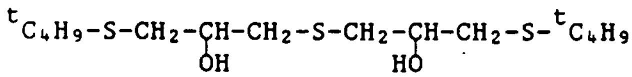

(III)

Reaktionsschema

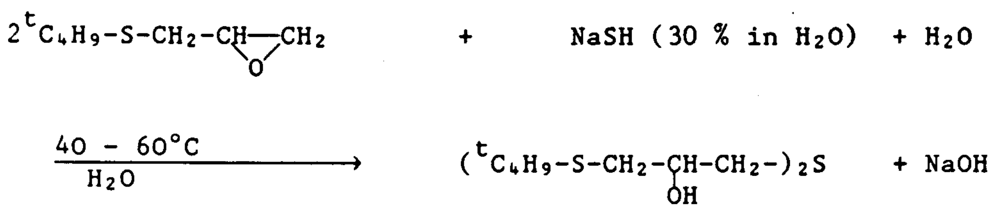


In einen Vierhalskolben werden zu 50 ml (0,268 Mol) technischer 30 %iger Natriumhydrogensulfid-Lauge - verdünnt mit 25 ml Wasser - unter Rühren 73,1 g (0,5 Mol) $^t$Butyl-glycidylthioether zugetropft (Dauer ca. 1 Stunde). Das sich dabei erwärmende Reaktionsgemisch wird durch Kühlung auf 40 - 50 °C gehalten. Anschliessend wird bei 50 - 60 °C noch 1 Stunde nachgerührt und der Endpunkt der Reaktion am Verschwinden des Glycidylthioether-Flecks im Dünnschichtchromatogramm kontrolliert.

Die Aufarbeitung erfolgt via Phasentrennung.

Die organische Phase ist das Reaktionsprodukt. Es wird mit ca. 20 ml verdünnter Natriumbicarbonatlösung nachgewaschen und nach scharfer Phasentrennung über CELITE filtriert und getrocknet.

Ausbeute: 77,6 g ≙ 95 % d.Th.; gelbe viskose Flüssigkeit;
Brechungsindex: $n_D^{20}$ : 1,5261
Sdp.: 180 °C / 0.5 mbar

Beispiel 2:

Herstellung von

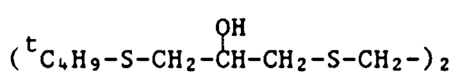

VI

Reaktionsschema

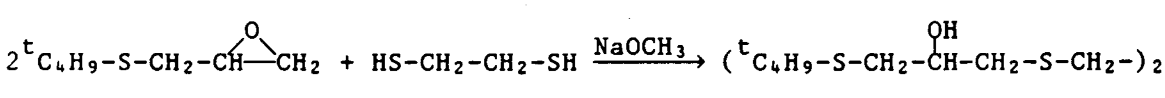


Arbeitsweise

In einem Zweihalskolben werden 18,8 g (0,2 Mol), 1,2-Dimercaptoäthan und 0,5 ml Natriummethylatlösung (30 %ig in Methanol) auf 60 °C erwärmt, das Heizbad entfernt und 61,4 g (0,42 Mol) $^t$Butylglycidylthioäther bei 60 - 70 °C unter Rühren innerhalb 45 Minuten eingetropft (exotherme Reaktion). Nach beendeter Zugabe wird das Gemisch noch 30 Minuten bei ca. 60 °C nachgerührt und im Rotationsverdampfer am Vakuum flüchtige Stoffe entfernt. Die Aufarbeitung erfolgt analog Beispiel 1.

Ausbeute: 100 % der Theorie; farblose Flüssigkeit.
Brechungsindex: $n_D^{20}$ : 1,5391

<u>Beispiel 3:</u>

Herstellung von

$$(^tC_4H_9-S-CH_2-\overset{OH}{\overset{|}{C}}H-CH_2-S-)_2 \qquad IV$$

<u>Reaktionsschema:</u>

$$2\,^tC_4H_9-S-CH_2-\overset{OH}{\overset{|}{C}}H-CH_2-SH + (CH_3)_2S = O$$

$$\xrightarrow[-(CH_3)_2S]{-H_2O} \quad (^tC_4H_9-S-CH_2-\overset{OH}{\overset{|}{C}}H-CH_2-S-)_2$$

36 g (0,5 mol) der Verbindung

$$^tC_4H_9-S-CH_2-\overset{OH}{\overset{|}{C}}H-CH_2-SH \;,$$

31,2 g (0,4 Mol) Dimethylsulfoxid und 150 ml Toluol werden in einem Reaktionsgefäss vereinigt und unter Rühren am Rückfluss während 4 Stunden wird 1,7 ml Reaktionswasser entfernt. Anschliessend wird ca. 100 ml Toluol/Dimethylsulfoxid bei Normaldruck abdestilliert, die organische Phase mit 50 ml Toluol versetzt, mit wässriger Natriumhydrogencarbonatlösung und anschliessend mit $H_2O$ gewaschen, dann getrocknet und eingeengt.

Ausbeute: 34,7 g ≙ 96,7 % der Theorie, gelbe Flüssigkeit: Brechungsindex $n_D^{20}$ : 1,5450

<u>Beispiel 4:</u>

<u>Verschleisschutz-Test:</u>

Die nach den Beispielen 1 bis 3 hergestellten Verbindungen III, IV und VI wird jede für sich einem zinkdithiophosphatfreien Motorenöl der SAE-Klasse 10 W-30 (Basis ISO VG 32-Mineralöl) mit der Bezeichnung AARG 45 in einer Konzentration von 1 Gew.-% zugemischt und die Proben dem sogenannten Cam und Tappet-Test (C + T) unterzogen.

Kurzbeschreibung der Prüfungsmethode C + T:

In einem geschlossenen Gehäuse rotiert ein Nocken aus Gusseisen, induktionsgehärtet auf 550 ÷ 580 KV (Vickers-Härte), gegen einen federbelasteten Stössel aus einsatzgehärtetem Stahl mit der Härte 800 ÷ 850 HV. Das Gehäuse ist bis zur Nockenachsmitte mit dem Prüföl gefüllt. Durch eingebaute Heizstäbe wird das Oel auf 100 °C aufgeheizt. Nach Erreichen der Solltemperatur wird die Spitzenkraft am Nocken durch Nachstellung der Feder auf 1000 N eingestellt und die Prüfung bei einer Nockendrehzahl von 1500 1/Min. begonnen. Nach einer Stunde Laufzeit wird der Verschleiss gemessen. Ist der Summenverschleiss von Nockenspitze und Stössel kleiner als 0,25 mm, so wird die Spitzenkraft um 100 N erhöht und der Test bei erhöhter Nockenkraft um 1 weitere Stunde fortgesetzt, solange bis der Verschleiss grösser als 0,25 mm ist oder die Spitzenkraft 2000 N erreicht hat.

Das Ergebnis wird als Schadenskraftstufe angegeben, oder, wenn kein Verschleiss erkennbar war, als grösser 2000 registriert.

In Tabelle 1 sind die ermittelten Werte aufgeführt, wobei ein höherer Zahlenwert (in N) einen verbesserten Verschleisschutz bedeutet.

Tabelle 1

| | C + T |
|---|---|
| | N |
| Basisöl AARG 45 | 1000 |
| + 1 % Verbindung der Formel III (Beispiel 1) | 1700 |
| + 1 % Verbindung der Formel IV (Beispiel 2) | 1800 |
| + 1 % Verbindung der Formel VI (Beispiel 3) | 1900 |

Beispiel 5:

Test auf Stabilisierung gegen Oxidation

Die Verbindungen hergestellt nach Beispielen 1 und 2 (Additive III und VI) werden dem TFOUT-Test unterzogen.

(TFOUT-Test: Thin-Film Oxygen Uptake Test, National Bureau of Standards (NBS), Washington D.C. USA)

Dieser Test ist eine modifizierte Version des "Rotary Bomb Oxidationstests für Mineralöle" (RBOT) (ASTM D 2272). Er wird genau beschrieben in "C.S. Ku und S.M. Hsu, A Thin-Film Oxygen Uptake Test for the Evaluation of Automotive Crankcase Lubricants, Lubrication Engineering, Vol. 40 (2), 75-83 (1984)". Beim TFOUT-Test wird das zu prüfende Oel (1,5 g) in einem Glas, das sich in einem Stahlbehälter (Bombe) befindet, in Gegenwart von 2 % Wasser, einer flüssigen oxidierten, nitrierten Fraktion eines Motorenbenzins als Katalysator (4 % Einsatzkonzentration) und eines flüssigen Metallnaphtenates als weiterem Katalysator (4 % Einsatzkonzentration) (das Wasser und die beiden flüssigen Katalysatorsubstanzen sind unter der No. Standard Reference Material 1817 vom National Bureau of Standards (NBS) mit Analysen-Zertifikat erhältlich) bei einem Sauerstoffdruck von 6,3 bar (90 psi) beaufschlagt (Sauerstoffvolumen 65 ml). Die Bombe rotiert in Schräglage mit 100 U/Min. in einem Flüssigkeitsbad von 160 °C. Die Schräglage und die Rotation bewirken eine filmartige Verteilung des Oeles auf der Mantelfläche des Glasbehälters.

Nach einer gewissen Induktionszeit beginnt das Oel unter diesen Bedingungen zu oxidieren. Die Oxidation bewirkt einen Sauerstoffverbrauch, der in einem deutlichen Druckabfall sichtbar wird. Die Zeit bis zum Beginn der Oxidation, die sogenannte Induktionszeit, wird als Mass für die Oxidationsstabilität genommen.

Als Testöl dient ein Motorenöl auf Mineralölbasis ISO VG 32, enthaltend ein Additiv-Paket Oloa 4140 C (Orogil), und einen Viskositätsindexverbesserer (Olefin-Copolymer). Die Viskosität entspricht 15W-50 und der Zinkdithiophosphatgehalt der Additive führt zu einem Analysenwert von 0,067 % Zn und 0,06 % P im Oel.

Die in der untenstehenden Tabelle 2 angegebenen Resultate bedeuten die Zeit (in Minuten) bis zum Knick des Druck/Zeit-Diagramms.

Lange Zeiten entsprechen guter Stabilisatorwirksamkeit. Konzentration der Additive III und VI: 0,75 Gew.-%, bezogen auf das Oel.

Tabelle 2

| TFOUT | min |
|---|---|
| Basisöl | 68 |
| + 0,75 Gew.% der Verbindung III gemäss Beispiel 1 | 275 |
| + 0,75 Gew.% der Verbindung VI gemäss Beispiel 2 | 272 |

Beispiel 6:

Test auf Verträglichkeit mit Dichtungsmaterialien

Geprüft wird nach der Volkswagenwerk-Methode VW-Sealtest, P-VW 3334, bei 150 °C und bei 96 h Testdauer. Die bewerteten Parameter sind die Aenderung des Moduls 1/3 (bei 1/3 Längung), die Aenderung der Reissfestigkeit und die Aenderung der Reissdehnung.

Die Prüfung eines Testmaterials des Types FKM (Fluor-Kautschuk) in einem Motorenöl enthaltend entweder die Verbindung der Formel III oder eine Verbindung der Formel VI bringt keine signifikante Aenderung des Zustandes der Dichtungen bezüglich Reissdehnung und Veränderung der Zugspannung gegenüber dem Motorenöl ohne die Zusätze nach Formel III und VI.

Beispiel 7:

Test auf Korrosionsverhalten gegenüber Kupfer

Es wird der Petter CEC L-O2-A-78-Test angewendet. Nach 36 Stunden werden die Werte für den Cu-Lager-Gewichtsverlust anhand einer Verbindung der Formel III und einer Verbindung der Formel VI, sowohl in Phosphor-freiem als auch in Phosphor-haltigem (P = 0,06 %) Oel geprüft. Die Werte des Gewichtsverlustes sind kleiner als 25 mg. Somit sind die Anforderungen der Klassen CCMC G1, G2 und G3 erfüllt.

**Patentansprüche**

**Patentansprüche für folgende Vertragsstaaten : BE, DE, FR, GB, IT, NL**

**1.** Verbindungen der Formel

$$R^1-S-CH_2-\underset{OH}{CH}-CH_2-Q-CH_2-\underset{OH}{CH}-CH_2-S-R^2 \qquad (I) ,$$

wobei Q -S-, -S-S-, $-S-CH_2-S-$ oder $-S-CH_2-CH_2-S-$ bedeutet,
und
worin $R^1$ und $R^2$ tert.-$C_4$- bis tert.-$C_7$-Alkyl bedeuten.

**2.** Verbindungen der Formel I nach Anspruch 1, worin $R^1$ und $R^2$ tert.-Butyl bedeuten.

**3.** Verbindung nach Anspruch 1 der Formel

$$\text{tert.-Butyl-S-CH}_2\text{-}\underset{\mathrm{OH}}{\mathrm{CH}}\text{-CH}_2\text{-S-CH}_2\text{-}\underset{\mathrm{OH}}{\mathrm{CH}}\text{-CH}_2\text{-S-tert.-Butyl} \quad \text{(III)},$$

$$\text{tert.-Butyl-S-CH}_2\text{-}\underset{\mathrm{OH}}{\mathrm{CH}}\text{-CH}_2\text{-S-S-CH}_2\text{-}\underset{\mathrm{OH}}{\mathrm{CH}}\text{-CH}_2\text{-S-tert.-Butyl} \quad \text{(IV)},$$

$$\text{tert.-Butyl-S-CH}_2\text{-}\underset{\mathrm{OH}}{\mathrm{CH}}\text{-CH}_2\text{-S-CH}_2\text{-S-CH}_2\text{-}\underset{\mathrm{OH}}{\mathrm{CH}}\text{-CH}_2\text{-S-tert.-Butyl} \quad \text{(V)},$$

$$\text{tert.-Butyl-S-CH}_2\text{-}\underset{\mathrm{OH}}{\mathrm{CH}}\text{-CH}_2\text{-S-CH}_2\text{-CH}_2\text{-S-CH}_2\text{-}\underset{\mathrm{OH}}{\mathrm{CH}}\text{-CH}_2\text{-S-tert.-Butyl} \quad \text{(VI)},$$

**4.** Zusammensetzung enthaltend
a) eine funktionelle Flüssigkeit aus der Reihe der Schmierstoffe, Hydraulikflüssigkeiten und Metallbearbeitungsflüssigkeiten und
b) wenigstens eine Verbindung der Formel I nach Anspruch 1.

**5.** Zusammensetzung nach Anspruch 4 enthaltend
a) ein Schmieröl aus der Reihe der Mineralöle, der synthetischen Oele oder der Mischungen davon, und
b) wenigstens eine Verbindung der Formel I.

**6.** Zusammensetzung nach Anspruch 5, worin das Schmieröl ein Oel für Verbrennungskraftmaschinen nach dem Diesel- oder Ottoprinzip ist.

**7.** Zusammensetzung nach Anspruch 5, der API-Klassifikation SF, SG, CD und/oder CE oder der CCMC-Klassen G 1, G 2, G 3, D 1, D 2, D 3 und/oder PD 1.

**8.** Verfahren zur Verminderung des Verschleisses in Verbrennungskraftmaschinen nach dem Diesel- oder Ottoprinzip, insbesondere an den Nocken der Nockenwellen und an den Stösseln des Ventiltriebes, dadurch gekennzeichnet, dass der Schmierölkreislauf der Verbrennungskraftmaschine mit einer Schmieröl-Zusammensetzung nach Anspruch 5 betrieben wird.

**9.** Verfahren zur Vermeidung der Desaktivierung des einer benzingetriebenen Verbrennungskraftmaschine nachgeschalteten Abgaskatalsysators, dadurch gekennzeichnet, dass der Schmierölkreislauf der Verbrennungskraftmaschine mit einer Schmieröl-Zusammensetzung nach Anspruch 5 betrieben wird.

**10.** Verwendung der Verbindungen der Formel I nach Anspruch 1 als Vielzweck-Additive für Schmierstoffe.

**11.** Verwendung nach Anspruch 10 als Kochdruck- und Verschleissschutzadditive für Schmierstoffe, Hydraulikflüssigkeiten oder Metallbearbeitungsflüssigkeiten.

**Patentansprüche für folgenden Vertragsstaat : ES**

**1.** Zusammensetzung enthaltend
a) eine funktionelle Flüssigkeit aus der Reihe der Schmierstoffe, Hydraulikflüssigkeiten und Metallbearbeitungsflüssigkeiten und
b) wenigstens eine Verbindung der Formel I

$$R^1\text{-S-CH}_2\text{-}\underset{\mathrm{OH}}{\mathrm{CH}}\text{-CH}_2\text{-Q-CH}_2\text{-}\underset{\mathrm{OH}}{\mathrm{CH}}\text{-CH}_2\text{-S-}R^2 \quad \text{(I)},$$

worin $R^1$ und $R^2$ tert.-$C_4$- bis tert.-$C_7$-Alkyl bedeuten und Q -S-, -S-S-, -S-$CH_2$-S- oder -S-$CH_2$-$CH_2$-S- bedeutet.

**2.** Zusammensetzung nach Anspruch 1 enthaltend
a) ein Schmieröl aus der Reihe der Mineralöle, der synthetischen Oele oder der Mischungen davon, und
b) wenigstens eine Verbindung der Formel I.

**3.** Zusammensetzung nach Anspruch 1, enthaltend wenigstens eine Verbindung der Formel I, worin $R^1$ und $R^2$ tert.-Butyl bedeuten.

**4.** Zusammensetzung nach Anspruch 1, enthaltend wenigstens eine Verbindung der Formeln

tert.-Butyl-S-$CH_2$-CH-$CH_2$-S-$CH_2$-CH-$CH_2$-S-tert.-Butyl (III),
OH OH

tert.-Butyl-S-$CH_2$-CH-$CH_2$-S-S-$CH_2$-CH-$CH_2$-S-tert.-Butyl (IV),
OH OH

tert.-Butyl-S-$CH_2$-CH-$CH_2$-S-$CH_2$-S-$CH_2$-CH-$CH_2$-S-tert.-Butyl (V),
OH OH

tert.-Butyl-S-$CH_2$-CH-$CH_2$-S-$CH_2$-$CH_2$-S-$CH_2$-CH-$CH_2$-S-tert.-Butyl (VI),
OH OH

**5.** Zusammensetzung nach Anspruch 2, worin das Schmieröl ein Oel für Verbrennungskraftmaschinen nach dem Diesel- oder Ottoprinzip ist.

**6.** Zusammensetzung nach Anspruch 2, der API-Klassifikation SF, SG, CD und/oder CE oder der CCMC-Klassen G 1, G 2, G 3, D 1, D 2, D 3 und/oder PD 1.

**7.** Verfahren zur Verminderung des Verschleisses in Verbrennungskraftmaschinen nach dem Diesel- oder Ottoprinzip, insbesondere an den Nocken der Nockenwellen und an den Stösseln des Ventiltriebes, dadurch gekennzeichnet, dass der Schmierölkreislauf der Verbrennungskraftmaschine mit einer Schmieröl-Zusammensetzung nach Anspruch 2 betrieben wird.

**8.** Verfahren Zur Vermeidung der Desaktivierung des einer benzingetriebenen Verbrennungskraftmaschine nachgeschalteten Abgaskatalsysators, dadurch gekennzeichnet, dass der Schmierölkreislauf der Verbrennungskraftmaschine mit einer Schmieröl-Zusammensetzung nach Anspruch 2 betrieben wird.

**9.** Verwendung der Verbindungen der Formel I nach Anspruch 1 als Vielzweck-Additive für Schmierstoffe.

**10.** Verwendung nach Anspruch 9 als Hochdruck- und Verschleissschutzadditive für Schmierstoffe, Hydraulikflüssigkeiten oder Metallbearbeitungsflüssigkeiten.

**Claims**

**Claims for the following Contracting States : BE, DE, FR, GB, IT, NL**

**1.** A compound of the formula

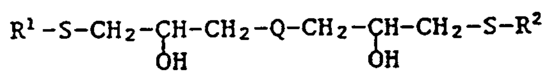

(I)

in which Q is -S-, -S-S-, $-S-CH_2-S-$ or $-S-CH_2-CH_2-S-$, and in which $R^1$ and $R^2$ are tert-$C_4$ to tert-$C_7$ alkyl.

**2.** A compound of the formula I according to claim 1, in which $R^1$ and $R^2$ are tert-butyl.

**3.** A compound according to claim 1 of the formula

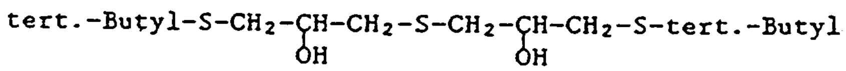

(III)

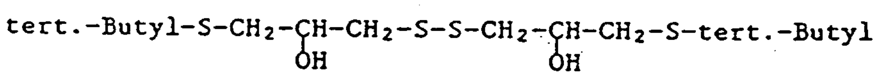

(IV)

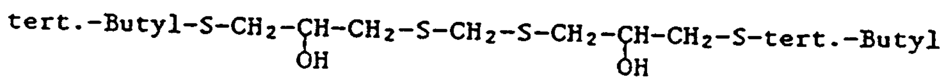

(V)

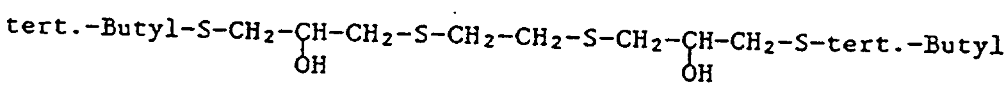

(VI)

**4.** A composition which comprises
a) a functional fluid from the series of lubricants, hydraulic fluids and metalworking fluids, and
b) at least one compound of the formula I according to claim 1.

**5.** A composition according to claim 4 which comprises
a) a lubricating oil from the series of mineral oils, synthetic oils or mixtures thereof, and
b) at least one compound of the formula I.

**6.** A composition according to claim 5, in which the lubricating oil is an oil for diesel or spark-ignition engines.

**7.** A composition according to claim 5, which conforms to API classification SF, SG, CD and/or CE or to CCMC categories G 1, G 2, G 3, D 1, D 2, D 3 and/or PD 1.

**8.** A process for reducing wear in diesel and spark-ignition engines, especially on the cams of the camshafts and on the tappets of the valve train, in which the lubrication system of the internal combustion engine is run on a lubricating oil having a composition according to claim 5.

**9.** A process for preventing the deactivation of the catalyst attached to the exhaust of a petrol engine, in which the lubrication system of the internal combustion engine is run on a lubricating oil having a composition according to claim 5.

**10.** Use of the compounds of the formula I according to claim 1 as multipurpose additives for lubricants.

**11.** Use according to claim 10 as extreme-pressure and antiwear additives for lubricants, hydraulic fluids or metalworking fluids.

**Claims for the following Contracting State : ES**

**1.** A composition which comprises
a) a functional fluid from the series of lubricants, hydraulic fluids and metalworking fluids, and
b) at least one compound of the formula I.

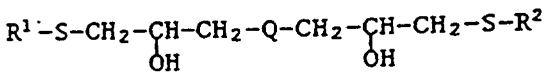

(I)

in which $R^1$ and $R^2$ are tert-$C_4$ to tert-$C_7$alkyl and Q is -S-, -S-S-, -S-$CH_2$-S- or -S-$CH_2$-$CH_2$-S-.

**2.** A composition according to claim 1 which comprises
a) a lubricating oil from the series of mineral oils, synthetic oils or mixtures thereof, and
b) at least one compound of the formula I.

**3.** A composition according to claim 1, which comprises at least one compound of the formula I, in which $R^1$ and $R^2$ are tert-butyl.

**4.** A composition according to claim 1, which comprises at least one compound of the formula

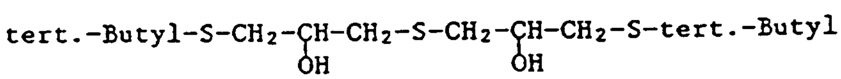

(III)

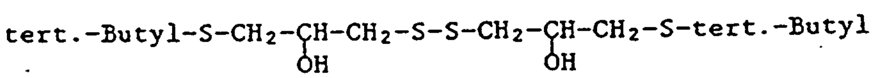

(IV)

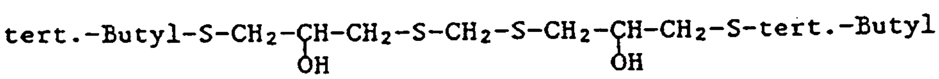

(V)

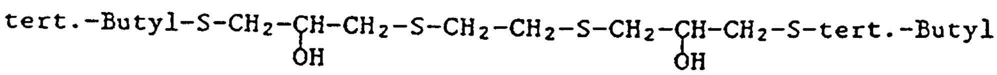

(VI)

**5.** A composition according to claim 2, in which the lubricating oil is an oil for diesel or spark-ignition engines.

**6.** A composition according to claim 2, which conforms to API classification SF, SG, CD and/or CE or to CCMC categories G 1, G 2, G 3, D 1, D 2, D 3 and/or PD 1.

**7.** A process for reducing wear in diesel and spark-ignition engines, especially on the cams of the camshafts and on the tappets of the valve train, in which the lubrication system of the internal combustion engine is run on a lubricating oil having a composition according to claim 2.

**8.** A process for preventing the deactivation of the catalyst attached to the exhaust of a petrol engine, in which the lubrication system of the internal combustion engine is run on a lubricating oil having a composition according to claim 2.

**9.** Use of the compounds of the formula I according to claim 1 as multipurpose additives for lubricants.

**10.** Use according to claim 9 as extreme-pressure and antiwear additives for lubricants, hydraulic fluids or metalworking fluids.

**Revendications**
**Revendications pour les Etats Contractants suivants : BE, DE, FR, GB, IT, NL**

**1.** Composés de formule

$$R^1-S-CH_2-\underset{OH}{CH}-CH_2-Q-CH_2-\underset{OH}{CH}-CH_2-S-R^2 \qquad (I),$$

dans laquelle Q signifie -S-, -S-S-, $-S-CH_2-S-$ ou $-S-CH_2-CH_2-S-$, et
$R^1$ et $R^2$ représentent un alkyle tertiaire en $C_4$-$C_7$.

**2.** Composés de formule I selon la revendication 1, dans laquelle $R^1$ et $R^2$ représentent un tert-butyle.

**3.** Composé selon la revendication 1, de formule

$$tert.\text{-}Butyl-S-CH_2-\underset{OH}{CH}-CH_2-S-CH_2-\underset{OH}{CH}-CH_2-S-tert.\text{-}Butyl \qquad (III),$$

$$tert.\text{-}Butyl-S-CH_2-\underset{OH}{CH}-CH_2-S-S-CH_2-\underset{OH}{CH}-CH_2-S-tert.\text{-}Butyl \qquad (IV),$$

$$tert.\text{-}Butyl-S-CH_2-\underset{OH}{CH}-CH_2-S-CH_2-S-CH_2-\underset{OH}{CH}-CH_2-S-tert.\text{-}Butyl \qquad (V),$$

$$tert.\text{-}Butyl-S-CH_2-\underset{OH}{CH}-CH_2-S-CH_2-CH_2-S-CH_2-\underset{OH}{CH}-CH_2-S-tert.\text{-}Butyl \qquad (VI)$$

**4.** Composition contenant
a) un fluide fonctionnel de la série des lubrifiants, des fluides hydrauliques et des fluides pour le traitement des métaux et
b) au moins un composé de formule I selon la revendication 1.

**5.** Composition selon la revendication 4, contenant
a) une huile lubrifiante de la série des huiles minérales, des huiles synthétiques ou de leurs mélanges, et
b) au moins un composé de formule I.

**6.** Composition selon la revendication 5, dans laquelle l'huile lubrifiante est une huile pour des machines à combustion interne fonctionnant selon le principe Diesel ou le principe d'allumage par étincelle.

**7.** Composition selon la revendication 5, de la classification API SF, SG, CD, et/ou CE ou des classes CCMC G 1, G 2, G 3, D 1, D 2, D 3 et/ou PD 1.

**8.** Procédé pour réduire l'usure dans des machines à combustion interne fonctionnant selon le principe Diesel ou le principe d'allumage par étincelle, en particulier au niveau des cames des arbres à cames et des poussoirs des mécanismes des soupapes, caractérisé en ce que le circuit d'huile lubrifiante de la machine à combustion interne fonctionne avec une composition d'huile lubrifiante selon la revendication 5.

**9.** Procédé pour réduire la désactivation d'un catalyseur intercalé en arrière d'une machine à combustion interne fonctionnant à l'essence, caractérisé en ce que le circuit d'huile lubrifiante de la machine à combustion interne fonctionne avec une composition d'huile lubrifiante selon la revendication 5.

**10.** Utilisation des composés de formule I selon la revendication 1 comme additifs universels pour des lubrifiants.

**11.** Utilisation selon la revendication 10 comme additifs haute pression et anti-usure pour des lubrifiants, des fluides hydrauliques et des fluides pour le traitement des métaux.

**Revendications pour l'Etat contractant suivant : ES**

**1.** Composition contenant
a) un fluide fonctionnel de la série des lubrifiants, des fluides hydrauliques et des fluides pour le traitement des métaux et
b) au moins un composé de formule

$$R^1-S-CH_2-\underset{OH}{CH}-CH_2-Q-CH_2-\underset{OH}{CH}-CH_2-S-R^2 \qquad (I)\ ,$$

dans laquelle $R^1$ et $R^2$ représentent un alkyle tertiaire en $C_4$-$C_7$ et Q signifie -S-, -S-S-, $-S-CH_2-S-$ ou $-S-CH_2-CH_2-S-$.

**2.** Composition selon la revendication 1, contenant
a) une huile lubrifiante de la série des huiles minérales, des huiles synthétiques ou de leurs mélanges, et
b) au moins un composé de formule I.

**3.** Composition selon la revendication 1, contenant au moins un composé de formule I dans laquelle $R^1$ et $R^2$ représentent un tert-butyle.

**4.** Composition selon la revendication 1, contenant au moins un composé de formule

$$\text{tert.-Butyl}-S-CH_2-\underset{OH}{CH}-CH_2-S-CH_2-\underset{OH}{CH}-CH_2-S-\text{tert.-Butyl} \qquad (III),$$

$$\text{tert.-Butyl}-S-CH_2-\underset{OH}{CH}-CH_2-S-S-CH_2-\underset{OH}{CH}-CH_2-S-\text{tert.-Butyl} \qquad (IV),$$

$$\text{tert.-Butyl}-S-CH_2-\underset{OH}{CH}-CH_2-S-CH_2-S-CH_2-\underset{OH}{CH}-CH_2-S-\text{tert.-Butyl} \qquad (V),$$

$$\text{tert.-Butyl}-S-CH_2-\underset{OH}{CH}-CH_2-S-CH_2-CH_2-S-CH_2-\underset{OH}{CH}-CH_2-S-\text{tert.-Butyl} \qquad (VI)$$

**5.** Composition selon la revendication 2, dans laquelle l'huile lubrifiante est une huile pour des machines à combustion interne fonctionnant selon le principe Diesel ou le principe d'allumage par étincelle.

6. Composition selon la revendication 2, de la classification API SF, SG, CD,et/ou CE ou des classes CCMC G 1, G 2, G 3, D 1, D 2, D 3 et/ou PD 1.

7. Procédé pour réduire l'usure dans des machines à combustion interne fonctionnant selon le principe Diesel ou le principe d'allumage par étincelle, en particulier au niveau des cames des arbres à cames et des poussoirs des mécanismes des soupapes, caractérisé en ce que le circuit d'huile lubrifiante de la machine à combustion interne fonctionne avec une composition d'huile lubrifiante selon la revendication 2.

8. Procédé pour réduire la désactivation d'un catalyseur intercalé en arrière d'une machine à combustion interne fonctionnant à l'essence, caractérisé en ce que le circuit d'huile lubrifiante de la machine à combustion interne fonctionne avec une composition d'huile lubrifiante selon la revendication 2.

9. Utilisation des composés de formule I selon la revendication 1 comme additifs universels pour des lubrifiants.

10. Utilisation selon la revendication 9 comme additifs haute pression et anti-usure pour des lubrifiants, des fluides hydrauliques et des fluides pour le traitement des métaux.